# EUROPEAN PATENT APPLICATION

(11) **EP 4 197 499 A1**
(43) Date of publication of application: **21.06.2023**
(21) Application number: 21215872.9
(22) Date of filing: 20.12.2021
(51) Int. Cl.: A61F 5/56, A61M 16/00, A61M 16/06

(54) **JAW POSITIONING**

(71) Applicant: Koninklijke Philips N.V., 5656 AG Eindhoven (NL)
(72) Inventor: KAHLERT, Joachim Johannes, Eindhoven (NL); VAN ZANTEN, Joyce, Eindhoven (NL); DOS SANTOS DA FONSECA, Pedro Miguel Ferreira, Eindhoven (NL); GRASSI, Angela, Eindhoven (NL)
(74) Representative: Philips Intellectual Property & Standards

(57) **Abstract**

In an embodiment, an apparatus (200) is described. The apparatus is for controlling positioning of a subject's (202) jaw (204). The apparatus comprises an expandable device (206) configured to expand to apply a force (F) on the subject's jaw in a direction of an anterior position (A) with respect to the subject's skull. The apparatus further comprises a mounting device (208) configured to hold the expandable device in proximity to the subject's jaw to facilitate application of the force on the subject's jaw.

## Description

### TECHNICAL FIELD OF THE INVENTION

The invention relates to apparatus, a method and systems for controlling positioning of a subject's jaw.

### BACKGROUND OF THE INVENTION

Obstructive sleep apnea (OSA) is a body position-dependent condition. In positional OSA, a patient's Apnea Hypopnea Index (AHI), Respiratory Disturbance Index (RDI) and/or Oxygen Desaturation Index (ODI) may be higher than an acceptable threshold, for example, when the patient is sleeping in the supine position. For patients treated by a positive airway pressure (PAP) therapy such as Continuous Positive Airway Pressure (CPAP) therapy or other therapies such as Bi-level PAP (BiPAP) therapy or Auto-PAP therapy, the PAP pressure may need to be relatively high to gain airway patency and rebalance the tidal volume. However, some patients may not tolerate such a high PAP pressure due to the perceived discomfort of the positive pressure. This perceived discomfort could contribute to arousals and may lower the PAP therapy adherence.

An advancement of the jawbone by an oral (in-mouth) device such as an oral Mandibular Advancement Device (MAD) is an example of an alternative therapy for OSA patients. Such oral devices may facilitate the advancement of the jawbone. However, such oral devices may be used where the patient has a healthy dentition since missing teeth, artificial teeth or certain other dental issues may prevent the use of an oral MAD. Further, a strong protrusion of the jawbone may create strong forces at the upper and lower teeth. This permanent force during the night can contribute to a dislocation of teeth and/or a loss of teeth by loosening in the jawbone and could, in the long-term, lead to a permanent deformation of the denture in some patients.

In OSA, an advancement of the jawbone by an oral MAD may be effective for mild and/or moderate OSA (e.g., 5< AHI <15). However, such an oral MAD may not be effective as a singular therapy in severe OSA (e.g., AHI>15). A combination therapy involving use of an oral MAD and PAP therapy may be a recommended approach to treat severe OSA.

However, wearing an oral MAD may not be comfortable or suitable for some patients. Further, PAP therapy can lead to discomfort in some patients.

### SUMMARY OF THE INVENTION

Certain aspects or embodiments described herein may relate to improving the management of certain conditions such as sleep disturbances and OSA. Certain aspects or embodiments may address problems relating to use of oral devices and/or PAP systems for managing such conditions.

In a first aspect of the invention, apparatus is described. The apparatus is for controlling positioning of a subject's jaw. The apparatus comprises an expandable device and a mounting device. The expandable device is configured to expand to apply a force on the subject's jaw in a direction of an anterior position with respect to the subject's skull. The mounting device is configured to hold the expandable device in proximity to the subject's jaw to facilitate application of the force on the subject's jaw.

Some embodiments relating to the first aspect and other aspects are described below.

In some embodiments, the expandable device is configurable in a first state and a second state. In the first state, the expandable device is unexpanded to allow the subject's jaw to move in a direction of a posterior position with respect to the subject's skull. In the second state, the expandable device is expanded to apply the force on the subject's jaw.

In some embodiments, the expandable device is configured to ensure that a diameter of the subject's airway is greater when the expandable device is in the second state than when the expandable device is in the first state.

In some embodiments, the expandable device is configured to prevent the subject's jaw from moving, under the effect of gravity, in a direction away from the anterior position.

In some embodiments, the expandable device is a non-oral device.

In some embodiments, the expandable device is fillable with a fluid to expand the expandable device.

In some embodiments, the expandable device is operatively coupled to a control system. The control system is configured to control the force applied on the jaw by the expandable device in response to the control system receiving an indication of a change in at least one of: an oxygen level of the subject; a gas flow rate of therapy air supplied to the subject; a position and/or orientation of the subject; and/or a sleep state of the subject.

In some embodiments, the control system is operatively coupled to a respiratory support system. The respiratory support system is configured to supply therapy gas to the subject via an interface associated with the apparatus. The respiratory support system is configured to control a pressure of the therapy gas supplied to the subject responsive to an indication of the subject's oxygen level provided to the control system by a sensing system.

In some embodiments, the respiratory support system is operatively coupled to the expandable device. The respiratory support system is further configured to supply gas to the expandable device for causing the expandable device to apply the force on the subject's jaw.

In some embodiments, the control system is configured to cause the expandable device to apply the force on the subject's jaw in response to receiving an indication that the pressure of the supplied therapy gas is too high for the subject.

In some embodiments, the control system is configured to cause the expandable device to apply the force on the subject's jaw in response to receiving an indication that the subject's oxygen level is too low.

In some embodiments, the mounting device is configured to position the expandable device behind the subject's jaw such that application of the force on the subject's jaw pushes the subject's jaw towards the anterior position.

In a second aspect, a patient support system is described. The patient support system comprises the apparatus of any of the first aspect or related embodiments. The patient support system further comprises a control system. The control system is configured to control the force applied on the jaw by the expandable device in response to the control system receiving an indication of a change in an oxygen level and/or sleep state of the subject. The patient support system further comprises a respiratory support system configured to supply therapy gas to the subject via an interface associated with the apparatus. The respiratory support system is configured to control a pressure of the therapy gas supplied to the subject responsive to an indication of the subject's oxygen level provided by a sensing system.

In a third aspect, a sleep support system is described. The sleep support system comprises the apparatus of any of the first aspect or related embodiments. The mounting device is configured to support the subject's chin during sleep.

An embodiment relating to the third aspect and other aspects is now described.

In some embodiments, the mounting device further comprises an upper lip support. The upper lip support is configured to permit movement of the jaw, in combination with the force applied by the expandable device, to adjust advancement of the subject's jaw in the direction of the anterior position.

In a fourth aspect, a method is described. The method controls positioning of a subject's jaw. The method comprises causing an expandable device to expand to apply a force on the subject's jaw in direction of an anterior position with respect to the subject's skull. The method further comprises holding the expandable device in proximity to the subject's jaw to facilitate application of the force on the subject's jaw.

Certain aspects or embodiments described herein may provide various technical improvements in terms of, for example, improving respiration quality in subjects such as patients with OSA and other conditions, providing non-intrusive support to a subject's jaw so that the subject's airway may be held open to improve the quality of the subject's respiration (e.g., while the subject is sleeping in a supine position), facilitating improved sleep in subject's with OSA and other conditions and/or providing a flexible approach to managing the subject's condition in an active and/or non-intrusive manner.

These and other aspects of the invention will be apparent from and elucidated with reference to the embodiment(s) described hereinafter.

### BRIEF DESCRIPTION OF DRAWINGS

Exemplary embodiments of the invention will now be described, by way of example only, with reference to the following drawings, in which:
Figures 1(a)-(b) are schematic drawings depicting how a subject's airway may be restricted by jaw displacement;
Figures 2(a)-(b) are schematic drawings of different views of an apparatus for controlling positioning of a subject's jaw according to an embodiment;
Figure 3 is a schematic drawing of an apparatus for use in a patient support system according to an embodiment;
Figure 4 is a schematic drawing of an apparatus for use in a sleep support system according to an embodiment;
Figure 5 refers to a method of controlling positioning of a subject's jaw according to an embodiment;
Figure 6 is a schematic drawing of a machine-readable medium for implementing various embodiments; and
Figure 7 is a schematic drawing of apparatus for implementing various embodiments.

### DETAILED DESCRIPTION OF THE EMBODIMENTS

A subject's jaw positioning may be affected by gravity when the subject is in certain positions such as the supine position (i.e., subject lying down and facing upwards) or by certain equipment that results in the application of force on the subject's jaw. In the supine position, or even in some non-supine positions, the jaw may be displaced (e.g., posteriorly displaced) with respect to the subject's skull. Such jaw displacement may restrict the subject's airway and lead to various issues such as snoring and/or reduced oxygen levels in the subject.

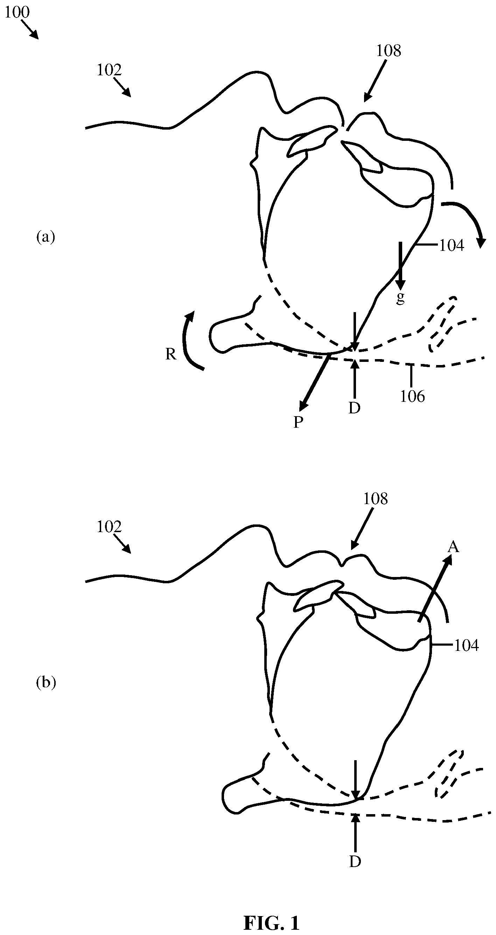

Figure 1 represents a scenario 100 depicting how gravity may affect a subject's 102 jaw 104 positioning while the subject 102 is in the supine position, and how this jaw positioning may affect the subject's airway 106. Figure 1 depicts a side cross-sectional view of the subject's head. In Figure 1(a), gravity, 'g', acting on the subject's jaw 104 causes the jaw 104 to rotate in the direction 'R' with respect to its joint and become displaced (i.e., towards a posterior position, 'P'). The rotational movement 'R' leads to part opening of the subject's mouth 108 with the subject's teeth moving away from their natural resting position of 'overbite' towards an 'open bite' position.

As depicted by Figure 1(a), the airway 106 is restricted (becomes narrower) in the oropharyngeal area to a diameter 'D' due to the posterior displacement of the jaw 104 in a direction towards the posterior position, P (i.e., the jaw 104 slips backwards from its natural resting position as shown by the arrow). A similar restriction effect may occur in other, non-supine, positions although the effect on the subject's airway 106 may vary. For example, if the subject's head leans to one side while their body remains on their back, the jaw 104 may undergo a certain amount of linear translatory motion leading to 'overjet' where the jaw 104 is horizontally displaced (i.e., a sideways displacement) from its natural resting position. Such linear translatory motion, potentially in combination with the rotation of the jawbone, may lead to a certain level of restriction in the airway 106.

The posterior displacement depicted by Figure 1(a) may also occur when certain equipment such as the straps of an oronasal mask (not shown here but described below) are fixed too tightly to the subject 102.

Figure 1(b) depicts the subject 102 of Figure 1(a) but with their jaw 104 in its natural resting position, in which the jaw 104 is in an anterior position, 'A' with respect to the subject's skull. As depicted, the subject's mouth 108 is closed with their teeth in the 'overbite' position while the diameter, D, of the airway 106 is larger than in Figure 1(a). In other words, while the jaw 104 is in the anterior position 'A', the airway 106 may remain open and relatively unrestricted compared with when the jaw 104 is in the posterior position 'P' with respect to the subject's skull.

In some subjects, it may be sufficient to stabilize the jawbone in the natural resting position to prevent the jaw 104 from being posteriorly displaced. In some subjects, it may be advisable to use an oral (in-mouth) device such as an oral MAD to cause the subject's jaw 104 to advance/move away from the posterior position e.g., by a few millimeters to reduce the likelihood of the subject's airway 106 becoming restricted. Such an advancement may be tolerated by some, but not all, subjects.

An oral (in-mouth) MAD may act to prevent the posterior displacement effect. However, some oral MADs are made of stiff monolithic parts. Such devices may not allow an opening of the mouth, a yawning or teeth grinding (bruxism), which is typical for some subjects during sleep. Subjects using such oral MADs may complain about muscle strain in their jawbone muscles.

Further, due to the mechanical constraints of the oral MAD, the teeth may not be able rest on each other in the manner depicted by Figure 1(b). The oral MAD may turn the natural overbite into an open bite, similar to that depicted by Figure 1(a). This open bite increases the height of the oral cavity. In this increased oral cavity, it is more likely that the tongue rolls back, which can contribute to a tongue base collapse and may worsen the airway 106 obstruction.

Therefore, oral MADs may also be used in the treatment or alleviation of sleep related conditions such as snoring and sleep apnea. However, oral MADs can be uncomfortable or inappropriate for some subjects.

In PAP therapy, a detected reduction in oxygen levels may lead to a PAP system supplying therapy gas (e.g., comprising environment air (20% oxygen) or air enriched by supplementary oxygen and/or other gas constituents) at a higher pressure. For this reason, the effective pressure is often higher in some subjects when using oronasal, as compared with exclusively nasal (mask) therapy, due to the posterior displacement effect described above. The increased pressure of the therapy gas may be uncomfortable for some subjects. In some cases, the PAP therapy may be combined with use of an oral MAD to reduce the need for increasing the pressure of the PAP therapy gas in case of airway restriction due to jaw displacement. However, as already described, there may be circumstances where oral MADs are uncomfortable or inappropriate.

Some non-oral (external to the mouth) devices may be used to treat or alleviate conditions such as snoring and sleep apnea. Such non-oral devices may also be used to prevent mouth breathing during nasal PAP therapy, in particular during a patient habituation period.

An example non-oral device is a chin strap, which may help to keep the subject's mouth closed. A non-oral device such as a chin strap may balance the horizontal position of the jawbone (e.g., to prevent overjet and reduce posterior movement of the jawbone) and the vertical position of the jawbone (e.g., to prevent a transition from overbite to open bite). In particular, controlling the vertical position of the jawbone (i.e., a chin lift to close the mouth) may prevent an unintended opening of the mouth and a transition to oral breathing, which may be associated with snoring. While a chin strap may be able to keep the mouth closed, the forces created by the straps can move the jawbone posteriorly. Therefore, chin straps may cause an airway narrowing at the tongue base (especially in the supine position). In other words, some non-oral devices such as chin straps may still lead to reduced oxygen levels and/or cause other issues.

Various embodiments described below may address at least one such issue and/or any other related issues as referred to herein.

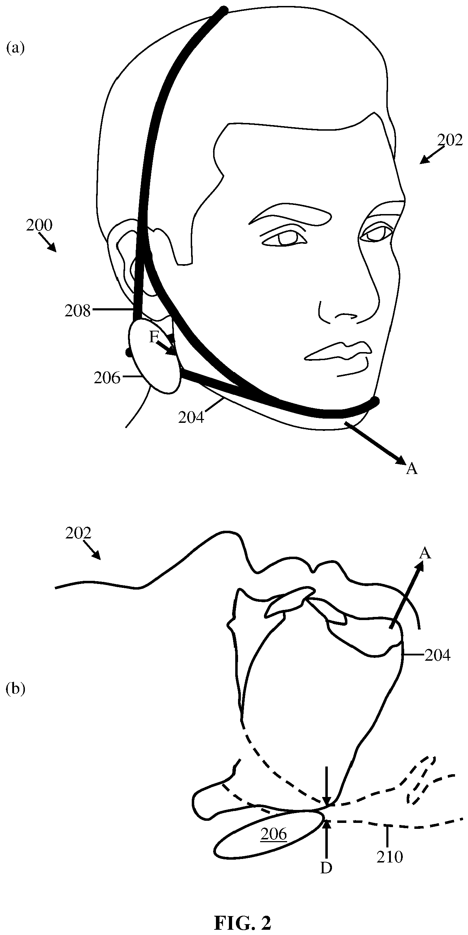

Figure 2(a) is a schematic drawing of an apparatus 200 for controlling positioning of a subject's 202 jaw 204 according to an embodiment. Figure 2(b) represents a side cross-sectional view of the subject 202 of Figure 2(a) depicting the positioning of part of the apparatus 200 with respect to the subject's head in order to position the subject's jaw 204 in the manner depicted by Figure 1(b).

The apparatus 200 comprises an expandable device 206 configured to expand to apply a force, 'F', on the subject's jaw 204 in a direction of an anterior position, 'A', with respect to the subject's skull. Thus, the force 'F' may be a vector acting on the jaw 204 in a direction that moves the jaw 204 towards the anterior position 'A'.

The apparatus 200 further comprises a mounting device 208 configured to hold the expandable device 206 in proximity to the subject's jaw 204 to facilitate application of the force on the subject's jaw 204.

The positioning of the expandable device 206 in proximity to the jaw 204 to facilitate application of the force on the subject's jaw 204 may be such that the expandable device 206 is in contact with the subject's skin to facilitate the application of the force due to the expansion. The contact point or area made by the expandable device 206 on the skin may depend on the properties of the expandable device 206 (such as its shape) and/or anatomical characteristics of the subject 202 (such as the size and weight of the jaw 204). The contact point or area may be behind the lower jaw 204 such as in the region of the skin over the ramus and/or angle of the mandible (jaw). Force could be applied to other parts of the jaw 204, providing the applied force results in the jaw 204 being moved towards (or prevented from moving away from) the anterior position. As described herein, the force may be applied on the "back" of the jaw 204, which could refer to a force applied in the region of the skin over the ramus and/or angle of the jaw.

In some embodiments, the mounting device 208 is configured to position the expandable device 206 behind (e.g., in the region of the ramus and/or angle) the subject's jaw 204 such that application of the force on the subject's jaw 204 pushes the subject's jaw 204 towards the anterior position (e.g., by virtue of causing the jaw 204 to rotate about its joint towards the overbite position).

The mounting device 208 is exemplary and depicts a possible way to position the expandable device 206 with respect to the subject's jaw 204. In this case, the mounting device 208 comprises a strap (such as a chin strap as described above) extending across the top of the subject's head and around the subject's chin under their lips. The strap further extends from the top of the subject's head, as well as from the chin, to the expandable device 206 positioned behind the back of the subject's lower jaw 204. The expandable device 206 is in a position that allows the expandable device 206 to apply the force, F, on the back of the jaw 204 when it is expanded so that the jaw 204 is held or moved towards the anterior position, A.

Although the mounting device 208 is depicted as being attached to the subject's head such as in the manner of a chin strap, other configurations are possible where the mounting device 208 is not attached to the subject's head. For example, the mounting device 208 may be a pillow upon which the subject's head is supported where the pillow incorporates the expandable device 206.

A first expandable device 206 is depicted in Figure 2. Although not visible in Figure 2, a second expandable device 206 may be provided in the same region on the other side of the subject's head. However, in various embodiments, there may be a single or multiple expandable devices 206 as part of the apparatus 200.

The expansion of the expandable device 206 may allow for the application of force, F, on the subject's jaw 204 to prevent the jaw 204 from moving in a direction towards posterior position and ensure that the jaw 204 is moved towards or is supported in the anterior position, A. Since the amount of weight applied by the jaw may vary by subject and/or by their position, the amount of expansion of the expandable device 206 needed to apply enough force on the jaw 204 may vary. The use of the expandable device 206 may allow a variable amount of force to be applied in dependence on the level of expansion, depending on the circumstances.

In some cases, the apparatus 200 and other embodiments described herein may be used to prevent the jaw 204 from falling backwards (i.e., by preventing linear translatory motion of the jawbone).

In some cases, the apparatus 200 and other embodiments described herein may be used to keep the mouth closed in its natural resting position (i.e., by preventing rotational motion of the jawbone when in the natural resting position or otherwise causing rotation of the jawbone so that the jawbone moves to its natural resting position).

In some cases, the apparatus 200 and other embodiments described herein may be used to advance/protrude the jaw to open the oropharyngeal airway to treat/alleviate: snoring, hypopnea (i.e., to lower the RDI, ODI) and/or mild/moderate OSA (i.e., to lower AHI) as a singular therapy without use of a positive airway pressure (PAP) therapy.

In some cases, the apparatus 200 and other embodiments described herein may be used to advance/protrude the jaw to permit a reduction of the therapy gas pressure (e.g., in a combination therapy of PAP therapy and jawbone stabilization and protrusion).

Thus, the apparatus 200 and other embodiments described may provide various technical improvements in terms of, for example, improving respiration quality in subjects such as patients with OSA and other conditions, providing non-intrusive support to a subject's jaw so that the subject's airway may be held open to improve the quality of the subject's respiration (e.g., while the subject is sleeping in a supine position), facilitating improved sleep in subject's with OSA and other conditions and/or providing a flexible approach to managing the subject's condition in an active and/or non-intrusive manner. Other technical improvements relating to the other embodiments are referred to below.

Some embodiments relating to features referred to in Figure 2, which may also be relevant to other embodiments as well, are now described.

In some embodiments, the expandable device 206 is configurable in a first state and a second state. In the first state, the expandable device 206 is unexpanded to allow the subject's jaw 204 to move in a direction of a posterior position with respect to the subject's skull. In the second state, the expandable device 206 is expanded to apply the force on the subject's jaw 204. By configuring the expandable device 206 between the two states, the advancement of the jaw 204 can be controlled depending on need (e.g., if more support is needed due to more weight being applied by the jaw 204 on the airway 210 when the subject 202 moves to the supine position, the expandable device 206 may be expanded into its second state, and vice versa).

In some embodiments, the expandable device 206 is configured to ensure that a diameter 'D' of the subject's airway 210 is greater when the expandable device 206 is in the second state than when the expandable device 206 is in the first state.

In some embodiments, the expandable device 206 is configured to prevent the subject's jaw 204 from moving, under the effect of gravity, in a direction away from the anterior position 'A'. For example, gravity may cause the jaw 204 to slip backwards, away from the anterior position, while the subject 202 is in the supine position. If the subject 202 is not in the supine position, gravity may still cause the jaw 204 to slip (to the side and/or backwards) in certain non-supine positions although the force component due to gravity may be lower such that a lower amount of force may be needed to support the jaw 204 in certain non-supine positions.

In some embodiments, the expandable device 206 is a non-oral device. Thus, the expandable device 206 may be relatively comfortable for the subject 202 and/or may avoid the abovementioned issues associated with oral devices while still providing the support for the jaw 204.

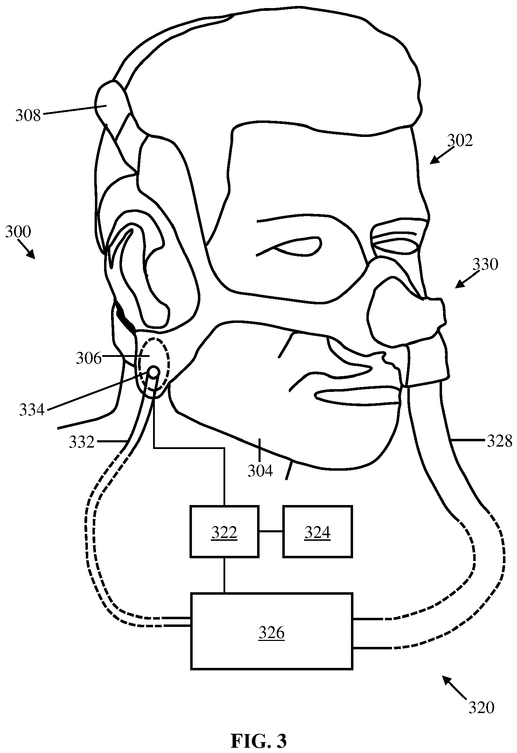

Figure 3 is a schematic drawing of an apparatus 300 for use in a patient support system 320 according to an embodiment. In this case, the patient support system 320 provides therapy gas for a subject 302 such as a patient. Reference signs for features of the apparatus 300 that are like or similar to corresponding features of the apparatus 200 are incremented by 100.

The apparatus 300 has similar functionality to the apparatus 200. However, additional functionality is provided for controlling the application of force on the subject's jaw 304 by the expandable device 306 of the apparatus 300.

In this regard, the patient support system 320 comprises a control system 322 for controlling the application of force by the expandable device 306. The control system 322 is communicatively coupled to the expandable device 306 to determine the level of expansion/force applied by the expandable device 306 (e.g., based on measurements from a pressure sensor associated with the expandable device 306, feedback system/control loop associated with expanding the expandable device 306, etc.). In some cases, the control system 322 may comprise processing circuitry and a machine readable medium storing instructions which, when executed by the processing circuitry, cause the processing circuitry to implemented the stated functionality of the control system 322.

The control system 322 is further communicatively coupled to a sensing system 324 of the patient support system 320. The sensing system 324 may determine various parameters associated with the subject 302 themselves or any equipment associated with the subject 302. For example, the sensing system 324 may implement an oxygen measurement system such as a photoplethysmogram (PPG) sensor to determine blood oxygen saturation levels (e.g., the ODI) in the subject 302. In some examples, the sensing system 324 may be able to infer oxygen levels based on parameters such as therapy gas pressure (where a high pressure may be indicative low blood oxygen levels). In some examples, the sensing system 324 may employ a positional/orientation sensor such as an accelerometer to determine the position or a change in position/orientation of the subject 302. Thus, the sensing system 324 may directly take measurements or indirectly infer various parameters about the subject 302, which may be used by the control system 322 to determine whether or not a force applied on the subject's jaw 304 is appropriate, or if it needs to change for any reason (e.g., to treat or alleviate a change in condition of the subject 302).

The control system 322 is further communicatively coupled to a respiratory support system 326 (such as a part of a PAP therapy system) of the patient support system 320. The respiratory support system 326 is configured to supply therapy gas to the subject 302 via a therapy gas supply hose 328 to an interface 330 associated with the subject 302. In this case, the interface 330 comprises a nasal mask which, in this case, is an integral part of the mounting device 308.

The mounting device 308 uses straps extending around the subject's head to hold the interface 330 over the subject's nose (e.g., to allow therapy gas to be supplied to the subject 302). As depicted by the dashed lines representing the outline of the expandable device 306, the mounting device 308 comprises the expandable device 306 in the region of the mounting device 308 that is in the proximity of the back of the subject's jaw 304. In some cases, the expandable device 306 may be an integral part of the mounting device 308 (e.g., attached to, inserted within or otherwise provided as a non-detachable part of the mounting device 308). In other cases, the expandable device 306 may be held by or otherwise supported by the mounting device 308 but may be detachable or otherwise readily removable from the mounting device 308, e.g., if the jaw support function is not needed at a certain time.

In this embodiment, the expandable device 306 comprises an inflatable cushion (fillable with gas such as air or therapy gas to inflate the cushion) integrated in the straps of the mounting device 308. The inflatable cushion may be based on a compressible balloon positioned/designed to avoid hampering the jaw 304 movement. An inflatable cushion may provide soft stabilization of the jaw 304 so that the jaw 304 can be advanced towards to or to its natural resting position.

In other embodiments, the expandable device 306 may comprise a balloon or a bladder fillable with liquid. Thus, any expandable device 306 that can be filled/expanded with a fluid such as liquid or gas to a pressure sufficient to exert a force on the jaw 304 that causes the jaw 304 to move towards (or be supported/prevented from moving away from) the anterior position may be used. Thus, in some embodiments, the expandable device 306 is fillable with a fluid such as gas or liquid to expand the expandable device 306. The level of force needed can be determined based on the weight of jaw 304 (which depends on the position of the subject 302) and the length of the cantilever (i.e., the distance between jawbone joint and position of the expandable device 306). The control system 322 may determine the pressure of fluid needed in the expandable device 306 to advance/support the jaw 304. For example, the parameter settings for inflating the expandable device 306 may be personalized to the anatomical properties of the subject 302, based on knowledge of the expansion/force application properties of the expandable device 306.

Although a separate inflation device such as a hand air pump, electric air pump, etc., could be provided for inflating the expandable device 306, in this embodiment, gas for inflating the expandable device 306 is supplied by the respiratory support system 326. In this regard, the patient support system 320 further comprises a gas supply hose 332 extending between the respiratory support system 326 and the expandable device 306, in which a gas supply port 334 (e.g., a hole/valve in the material of the expandable device 306) is formed to facilitate inflation/deflation of the expandable device 306. Thus, in this embodiment, the same equipment can be utilized for supplying therapy gas to the subject 302 while also facilitating the functionality of the expandable device 306. Although not depicted, in some embodiments, the gas supply hose 332 may pass through the control system 322, which may itself comprise a valve system to control when and for how long gas is to be supplied to/released from the expandable device 306. In some cases, such a valve system may be provided as part of the respiratory support system 326 itself.

In a combination therapy of PAP therapy and chin lifting/jaw 304 advancement, the patient support system 320 may independently adapt both the pressure of the therapy gas (i.e., the PAP titration pressure) and the gas for inflation of the expandable device 306. For example, there may be circumstances where the subject 302 needs to receive more oxygen, in which case the control system 322 may cause the respiratory support system 326 to increase the therapy gas pressure and/or inflate the expandable device 306. If the subject 302 finds the increased therapy gas pressure unpleasant, this may be detected/indicated to the control system 322, which may then cause a reduction in the therapy gas pressure and increase the support provided by the expandable device 306. Thus, the control system 322 may be useable for balancing the therapy gas supply and the force applied by the expandable device 306, depending on the circumstances at the time.

Some embodiments relating to features referred to in Figure 3, which may also be relevant to other embodiments as well, are now described.

In some embodiments, the expandable device 306 is operatively coupled to the control system 322. In such embodiments, the control system 322 is configured to control the force applied on the jaw 304 by the expandable device 306 in response to the control system 322 receiving an indication of a change. The change may be in respect of at least one of: an oxygen level of the subject 302; a gas flow rate of therapy air supplied to the subject 302; a position and/or orientation of the subject 302; and/or a sleep state (e.g., awake, asleep, which sleep phase the subject 302 is in, etc.) of the subject 302. In some embodiments, the indication may be provided by the sensing system 324 (e.g., the oxygen level, position/orientation and/or the sleep state may be indicated by the sensing system 324). In some embodiments, the indication may be provided by the respiratory support system 326 (e.g., the gas flow rate). In some cases, the sensing system 324 may be configured to detect various changes for example, via different sensors deployed to monitor different aspects of the subject 302 and/or the patient support system 320.

In some embodiments, the control system 322 is operatively coupled to the respiratory support system 326. In such embodiments, the respiratory support system 326 is configured to supply therapy gas to the subject 302 via the interface 330, which may be associated with the apparatus 300 by virtue of being part of the mounting device 308. The respiratory support system 326 is configured to control a pressure of the therapy gas supplied to the subject 302 responsive to an indication of the subject's oxygen level provided to the control system 322 by the sensing system 324 (e.g., such as a PPG sensor).

In some embodiments, the respiratory support system 326 is operatively coupled to the expandable device 306 (e.g., via the gas supply hose 332) and configured to supply gas to the expandable device 306 for causing the expandable device 306 to apply the force on the subject's jaw 304.

In some embodiments, the control system 322 is configured to cause the expandable device 306 to apply the force on the subject's jaw 304 in response to receiving an indication that the pressure of the supplied therapy gas is too high for the subject 302.

In some embodiments, the control system 322 is configured to cause the cause the expandable device 306 to apply the force on the subject's jaw 304 in response to receiving an indication that the subject's oxygen level is too low. Such an indication may be provided by the sensing system 324 (e.g., via a direct reading of the subject's oxygen level) and/or the respiratory support system 326 (e.g., via an indirect indication such as an increase in the pressure of the therapy gas being supplied).

By being able to control the force applied on the subject's jaw 304 in combination with being able to control the pressure of the therapy gas, a combination therapy may be provided based on controlling both of these parameters, which may reduce the need to rely solely on controlling the pressure of the therapy gas. For example, the use of the apparatus 300 may facilitate jaw advancement to reduce the need for an elevated therapy gas pressure, which the subject 302 may find uncomfortable. When provided in non-oral form, the expandable device 306 may be comfortable for the subject 302. It may also be possible that the jaw stability provided by the apparatus 300 may improve velopharyngeal patency. Thus, the apparatus 300 may help to minimize side effects and increase the likelihood of achieving a high therapy adherence. A combination therapy may facilitate improvements in various outcome parameters, such as reduction of effective pressure of therapy gas, reduction of residual AHI and/or increase in patient compliance. The combination therapy may be useful for subjects using oronasal masks where additional forces may be placed on the jaw by the mask itself.

The patient support system 320 may comprise the apparatus 200, 300 or any other apparatus according to other embodiments described herein. In some embodiments, the patient support system 320 comprises the control system 322, which may be configured to control the force applied on the jaw 304 by the expandable device 306 in response to the control system 322 receiving an indication of a change in an oxygen level and/or sleep state of the subject 302. In some embodiments, the patient support system 320 comprises the respiratory support system 326. Any embodiments relating to the control system 322 and/or the respiratory support system 326 may be implemented in the patient support system 320.

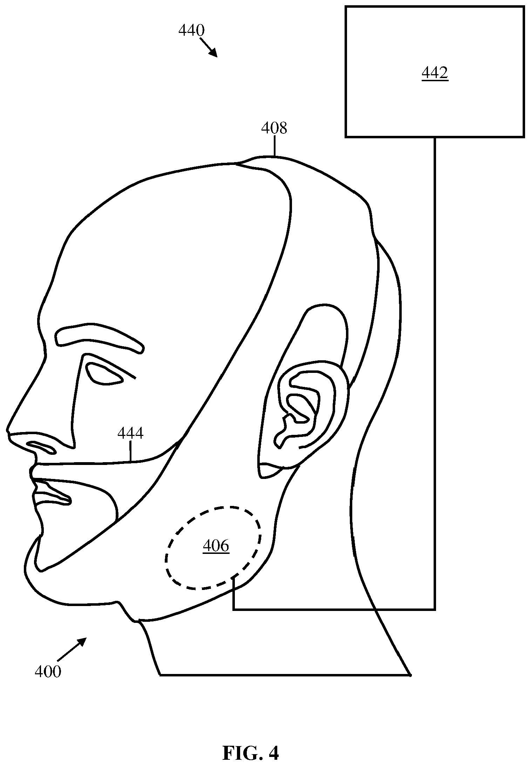

Figure 4 is a schematic drawing of an apparatus 400 for use in a sleep support system 440 according to an embodiment. Reference signs for features of the apparatus 400 that are like or similar to corresponding features of the apparatus 300 are incremented by 100. The apparatus 400 comprises an expandable device 406 (represented by the dashed line) and a mounting device 408. In this embodiment, the mounting device 408 is configured to support the subject's chin during sleep. As depicted, this could be in the form of a chin strap extending around the head and chin, similar to the design depicted by Figure 2. The provision of the expandable device 406 as part of the apparatus 400 may help to reduce the tendency for the jaw to slip backwards especially when the subject is in the supine position. While the chin strap design may be useful for some circumstances, it may not adapt to the subject's position. Thus, the expandable device 406 may improve jaw positioning to reduce snoring and/or the effects of OSA in respect of the apparatus 400, which may provide such benefits for the subject irrespective of their position.

Thus, the apparatus 400 may be useable by subjects that have the tendency to snore, which may be one of the signs for mild to moderate OSA. In some embodiments, equipment 442 such as the respiratory support system 326 (or a dedicated pump) can provide the fluid for expanding the expandable device 406. In some embodiments, other equipment such as the control system 322 and/or sensing system 324 may be used in conjunction with this equipment 442 in order to provide the functionality described.

In some embodiments, the mounting device 408 further comprises an upper lip support 444 configured to permit movement (e.g., displacement between the posterior and anterior positions) of the jaw, in combination with the force applied by the expandable device 406, to adjust advancement of the subject's jaw in the direction of the anterior position. The upper lip support 444 may comprise a further strap, connected to the mounting device 408. The upper lip support 444 may, when tensioned, apply a force on the subject's upper lip. In combination with the force applied by the expandable device 406, additional control may be provided over the positioning of the jaw with respect to the skull. For example, the force acting on the upper lip may, in combination with the force acting on the back of the jaw, help to provide rotation of the jaw towards the overbite position (e.g., to advance the jaw forwards with respect to the subject's skull).

Any of the features described in relation to the apparatus 200, 300, 400 may be included with various types of mounting devices 208, 308, 408. Examples of mounting devices 208, 308, 408 include chin supports, nasal masks, oronasal masks and pillows.

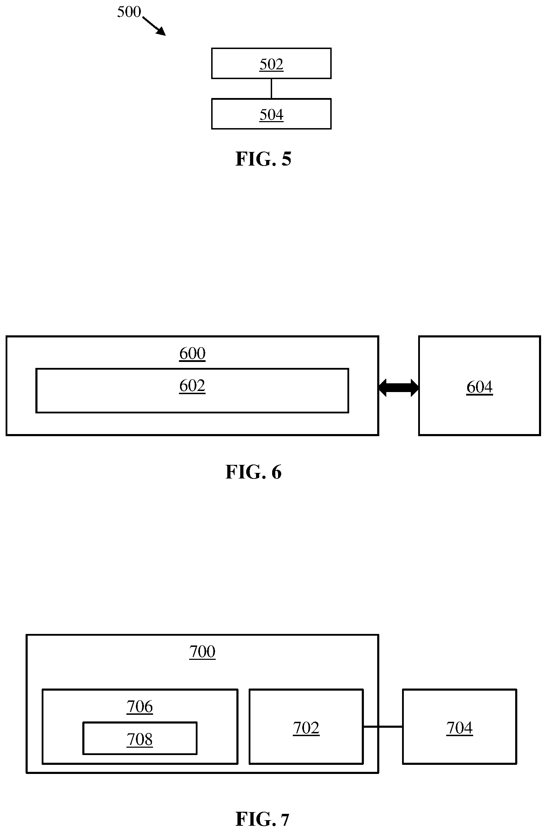

Figure 5 refers to a method 500 of controlling positioning of a subject's jaw according to an embodiment. The method 500 may implement a non-therapeutic method such as aiding sleep, reducing snoring, etc. The method 500 may be implemented by using various apparatus described herein such as the apparatus 200, 300, 400 and the related embodiments. Reference is made to Figures 2 and 3 in the description of the method 500. Further, the blocks of the method 500 may be performed in any order.

In this embodiment, the method 500 comprises, at block 502, causing an expandable device 206 to expand to apply a force on the subject's jaw 204 in direction of an anterior position with respect to the subject's skull. In some embodiments, the control system 322 may be used to cause the expandable device 306 to expand to apply the force.

The method 500 further comprises, at block 504, holding the expandable device 206 in proximity to the subject's jaw 204 to facilitate application of the force on the subject's jaw 204.

Any methods relating to the embodiments described above (e.g., in relation to the apparatus 200, 300, 400) may be implemented in conjunction with the method 500.

Figure 6 is a schematic drawing of a non-transitory machine-readable medium 600 for implementing the functionality of certain embodiments described herein. For example, certain functionality of the control system 322 of Figure 3 may be implemented by the machine-readable medium 600. The machine-readable medium 600 stores instructions 602 which, when executed by processing circuitry 604, cause the processing circuitry 604 to implement such functionality.

Figure 7 is a schematic drawing of apparatus 700 for implementing functionality of certain embodiments described herein. For example, certain functionality of the control system 322 of Figure 3 may be implemented by the apparatus 700.

The apparatus 700 comprises processing circuitry 702. The processing circuitry 702 is configured to communicate with an interface 704. The interface 704 may be any interface (wireless or wired) implementing a communications protocol to facilitate exchange of data with other devices such as the sensing system 324 and/or respiratory support system 326.

The apparatus 700 further comprises a machine-readable medium 706 (e.g., non-transitory or otherwise) storing instructions 708 which, when executed by the processing circuitry 702, cause the processing circuitry 702 to implement the functionality of certain embodiments described herein.

While the invention has been illustrated and described in detail in the drawings and foregoing description, such illustration and description are to be considered illustrative or exemplary and not restrictive; the invention is not limited to the disclosed embodiments.

One or more features described in one embodiment may be combined with or replace features described in another embodiment.

Embodiments in the present disclosure can be provided as methods, systems or as a combination of machine-readable instructions and processing circuitry. Such machine-readable instructions may be included on a non-transitory machine (for example, computer) readable storage medium (including but not limited to disc storage, CD-ROM, optical storage, flash storage, etc.) having computer readable program codes therein or thereon.

The present disclosure is described with reference to flow charts and block diagrams of the method, devices, and systems according to embodiments of the present disclosure. Although the flow charts described above show a specific order of execution, the order of execution may differ from that which is depicted. Blocks described in relation to one flow chart may be combined with those of another flow chart. It shall be understood that each block in the flow charts and/or block diagrams, as well as combinations of the blocks in the flow charts and/or block diagrams can be realized by machine readable instructions.

The machine-readable instructions may, for example, be executed by a general-purpose computer, a special purpose computer, an embedded processor, or processors of other programmable data processing devices to realize the functions described in the description and diagrams. In particular, a processor or processing circuitry, or a module thereof, may execute the machine-readable instructions. Thus, functional modules of apparatus and other devices described herein may be implemented by a processor executing machine readable instructions stored in a memory, or a processor operating in accordance with instructions embedded in logic circuitry. The term 'processor' is to be interpreted broadly to include a CPU, processing unit, ASIC, logic unit, or programmable gate array etc. The methods and functional modules may all be performed by a single processor or divided amongst several processors.

Such machine-readable instructions may also be stored in a computer readable storage that can guide the computer or other programmable data processing devices to operate in a specific mode.

Such machine-readable instructions may also be loaded onto a computer or other programmable data processing devices, so that the computer or other programmable data processing devices perform a series of operations to produce computer-implemented processing, thus the instructions executed on the computer or other programmable devices realize functions specified by block(s) in the flow charts and/or in the block diagrams.

Further, the teachings herein may be implemented in the form of a computer program product, the computer program product being stored in a storage medium and comprising a plurality of instructions for making a computer device implement the methods recited in the embodiments of the present disclosure.

Elements or steps described in relation to one embodiment may be combined with or replaced by elements or steps described in relation to another embodiment. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems.

## Claims

1. Apparatus (200) for controlling positioning of a subject's (202) jaw (204), the apparatus comprising:
an expandable device (206) configured to expand to apply a force (F) on the subject's jaw in a direction of an anterior position (A) with respect to the subject's skull; and
a mounting device (208) configured to hold the expandable device in proximity to the subject's jaw to facilitate application of the force on the subject's jaw.

2. The apparatus of claim 1, wherein the expandable device is configurable in a first state and a second state, wherein:
in the first state, the expandable device is unexpanded to allow the subject's jaw to move in a direction of a posterior position with respect to the subject's skull; and
in the second state, the expandable device is expanded to apply the force on the subject's jaw.

3. The apparatus of claim 2, wherein the expandable device (206) is configured to ensure that a diameter (D) of the subject's airway (210) is greater when the expandable device is in the second state than when the expandable device is in the first state.

4. The apparatus of any of claims 1 to 3, wherein the expandable device is configured to prevent the subject's jaw from moving, under the effect of gravity, in a direction away from the anterior position.

5. The apparatus of any of claims 1 to 4, wherein the expandable device is a non-oral device.

6. The apparatus (300) of any of claims 1 to 5, wherein the expandable device is fillable with a fluid to expand the expandable device.

7. The apparatus (300) of any of claims 1 to 6, wherein the expandable device (306) is operatively coupled to a control system (322) configured to control the force applied on the jaw by the expandable device in response to the control system receiving an indication of a change in at least one of:
an oxygen level of the subject;
a gas flow rate of therapy air supplied to the subject;
a position and/or orientation of the subject; and/or
a sleep state of the subject.

8. The apparatus of claim 7, wherein the control system is operatively coupled to a respiratory support system (326) configured to supply therapy gas to the subject via an interface (330) associated with the apparatus, and wherein the respiratory support system is configured to control a pressure of the therapy gas supplied to the subject responsive to an indication of the subject's oxygen level provided to the control system by a sensing system (324).

9. The apparatus of claim 8, wherein the respiratory support system is operatively coupled to the expandable device and configured to supply gas to the expandable device for causing the expandable device to apply the force on the subject's jaw.

10. The apparatus of any of claims 8 to 9, wherein the control system is configured to:
cause the expandable device to apply the force on the subject's jaw in response to receiving an indication that the pressure of the supplied therapy gas is too high for the subject; and/or
cause the expandable device to apply the force on the subject's jaw in response to receiving an indication that the subject's oxygen level is too low.

11. The apparatus of any of claims 1 to 10, wherein the mounting device is configured to position the expandable device behind the subject's jaw such that application of the force on the subject's jaw pushes the subject's jaw towards the anterior position.

12. A patient support system (320), comprising:
the apparatus (200, 300) of any of claims 1 to 11;
a control system (322) configured to control the force applied on the jaw by the expandable device in response to the control system receiving an indication of a change in an oxygen level and/or sleep state of the subject; and
a respiratory support system (326) configured to supply therapy gas to the subject via an interface associated with the apparatus, and wherein the respiratory support system is configured to control a pressure of the therapy gas supplied to the subject responsive to an indication of the subject's oxygen level provided by a sensing system (324).

13. A sleep support system (440), comprising:
the apparatus (200, 300, 400) of any of claims 1 to 11, wherein the mounting device (408) is configured to support the subject's chin during sleep.

14. The sleep support system of claim 13, wherein the mounting device further comprises an upper lip support (444) configured to permit movement of the jaw, in combination with the force applied by the expandable device (406), to adjust advancement of the subject's jaw in the direction of the anterior position.

15. A method (500) of controlling positioning of a subject's jaw, the method comprising:
causing (502) an expandable device to expand to apply a force on the subject's jaw in direction of an anterior position with respect to the subject's skull; and
holding (504) the expandable device in proximity to the subject's jaw to facilitate application of the force on the subject's jaw.
